# EUROPEAN PATENT APPLICATION

(11) **EP 4 623 955 A1**
(43) Date of publication of application: **01.10.2025**
(21) Application number: 24166615.5
(22) Date of filing: 27.03.2024
(51) Int. Cl.: A61M 5/20, A61M 5/24, A61M 5/315

(54) **A DRUG DELIVERY INJECTION DEVICE AND SYSTEM**

(71) Applicant: Vox Medical ApS, 4200 Slagelse (DK)
(72) Inventor: Geertsen, Thomas, 4200 Slagelse (DK)
(74) Representative: Chas. Hude A/S

(57) **Abstract**

Disclosed is an injection device for delivering set doses of a drug from a cartridge. The injection device comprises a main body. The injection device comprises a cartridge holder configured for holding the cartridge. The injection device comprises a piston rod assembly comprising a piston rod. The injection device comprises a lock assembly. The loc assembly is configured for releasably locking the cartridge holder to the main body. The injection device comprises a drive mechanism. The drive mechanism is configured for driving the lock assembly to unlock the cartridge holder from the main body. The drive mechanism comprises at least one electric motor.

## Description

### TECHNICAL FIELD

The present invention relates to a drug delivery injection device or an injection device for delivering set doses of a drug from a cartridge. The invention further relates to a system comprising one or more external devices for use with said injection device.

### BACKGROUND

User operated injection devices for delivering set doses of a drug from a cartridge are often designed to make drug administration from a cartridge easier and more convenient for the user. Certain injection devices are disposable, while others are reusable devices that can be used for multiple injections by replacing the cartridge and/or the needle. The choice between a reusable and a disposable injection device depends on factors such as frequency of use, cost considerations, environmental concerns, ease of use, and individual preferences. Usually, only one reusable injection device is needed for leaving the house, making it easy to carry in a pocket or bag. Users can conveniently take the injection device wherever they go, whether at work, school, or while traveling. Furthermore, because the main body of the injection device remains the same, users can rely on consistent performance and familiarity with the device's operation. However, while reusable injection devices offer several benefits, they also come with some potential disadvantages. The main body of a reuseable injection device typically includes a mechanism for dose delivery and various other components, which can make the device larger and heavier than disposable injection devices. Furthermore, a reusable device requires unlocking and locking the device to insert the cartridge, which may be difficult for some users with impaired strength. Furthermore, because reusable injection devices are designed for multiple uses, they may experience wear and tear over time. Parts such as the dose dial or mechanism for dose delivery may degrade with frequent use, requiring eventual replacement of the entire injection device and potentially risking malfunctioning and/or that the injection device provides an incorrect dose. Finally, because the user may receive more than one dose from the same cartridge, the risk of user error in drug administration may be greater than for disposable devices. There is a need for an improved injection device, especially an improved reusable injection device.

### SUMMARY

In accordance with the present disclosure, the above-mentioned and other disadvantages are obtained by the disclosed injection device and system.

Disclosed is an injection device for delivering set doses of a drug from a cartridge. The injection device comprises a main body. The injection device comprises a cartridge holder configured for holding the cartridge. The injection device comprises a piston rod assembly comprising a piston rod. The injection device comprises a lock assembly. The lock assembly is configured for releasably locking the cartridge holder to the main body. The injection device comprises a drive mechanism. The drive mechanism is configured for driving the lock assembly to unlock the cartridge holder from the main body. The drive mechanism comprises at least one electric motor.

The injection device or drug delivery device is configured for delivering set doses of a drug or medicament from a cartridge. The injection device is configured for being used by a user. The injection device may be configured for being a reusable injection device, but may alternative be configured for being a disposable injection device. Thus, the disclosed subject matter is not limited to reusable injection devices. The cartridge may be provided with a needle attached or a needle may be attached to the cartridge by the user. The injection device comprises a main body. The injection device comprises a cartridge holder configured for holding the cartridge. The injection device comprises a piston rod assembly comprising a piston rod. The piston rod assembly may be configured for facilitating delivering set doses of the drug from the cartridge, by pushing or forcing the drug out of the cartridge through the needle. The injection device comprises a lock assembly. The lock assembly is configured for releasably locking the cartridge holder to the main body. In this regard, locked is taken to mean securely attached, such as preventing unintentional detachment or separation. Further in this regard, releasably locked is taken to mean that the locking can be intentionally undone without causing damage to any components. Thus, the lock assembly is configured for locking the cartridge holder to the main body as well as unlocking the cartridge holder from the main body. The injection device comprises a drive mechanism. In this regard, the drive is defined as to send, cause and/or guide a movement. The drive mechanism is configured for driving the lock assembly to unlock the cartridge holder from the main body. The drive mechanism comprises at least one electric motor.

It is an advantage that an improved injection device is provided. The injection device as disclosed provides that the at least one electric motor is driving unlocking the cartridge holder from the main body, thereby providing an injection device, such as a reusable injection device, that is easy to use and does not require strength to unlock.

It should be noted that the use of the terms lock assembly, piston rod assembly and drive mechanism is used for explanatory purposes and should not be considered limiting. Any feature mentioned as comprised in or provided in any of the lock assembly, piston rod assembly and drive mechanism, respectively, thereby also comprised in or provided in the injection device.

In some embodiments, an longitudinal axis of the injection device runs lengthwise through a centre of the injection device. Thus, the longitudinal axis may be a central axis.

In some embodiments, the drive mechanism is configured for driving the lock assembly to unlock the cartridge holder from the main body by driving the piston rod assembly to engage with the lock assembly. Thus, the drive mechanism may be configured for moving or applying force to the piston rod assembly, such as along the longitudinal axis. Thereby, the piston rod assembly may engage with the lock assembly.

In some embodiments, the at least one electric motor may be any electric motor in the injection device, thus not necessary a same motor as for driving the piston rod assembly to engage with the cartridge to deliver set doses of the drug. Alternatively, in some embodiments, the drive mechanism is further configured for driving the piston rod assembly to engage with the cartridge to deliver set doses of the drug. Thus, the drive mechanism with the at least one electric motor may be configured for driving the lock assembly as well as the piston rod assembly for engaging with the cartridge. Thereby, the same at least one electric motor is providing that the cartridge holder is unlocked from the main body and is providing that drug is delivered from the cartridge.

In some embodiments, the lock assembly comprises one or more first male components and one or more first female components. The one or more first male components may be configured for engaging with the one or more first female components. In some embodiments, the lock assembly further comprises a rotary lock. Thus, in other words, the injection device may comprise the rotary lock. The one or more first male components may be provided at the rotary lock and the one or more first female components may be provided at the cartridge holder, or vice versa. The one or more first male components or one or more first female components provided at the cartridge holder may be provided at an inside surface, at an outside surface or at an boundary/edge between the inside and outside surface of the cartridge holder.

In some embodiments, the piston rod and the rotary lock comprises external threads and internal threads, respectively. The piston rod assembly engaging with the lock assembly may comprise rotating the rotary lock. The rotary lock may rotate around the longitudinal axis.

In some embodiments, the lock assembly further comprises a rotary lock spring configured for providing energy to support releasably locking the cartridge holder to the main body. Thus, in other words, the injection device may comprise the rotary lock spring. The rotary lock spring may be a torsion-spring, such as a helical torsion spring. Thus, the rotary spring may be a mechanical spring configured for exerting torque or twisting force when it is twisted. Due to the one or more first male components provided at the rotary lock and the one or more first female components provided at the cartridge holder, or vice versa, the rotary lock spring may be configured for being twisted or rotated as the one or more first male components are engaging with the one or more first female components, the rotary lock spring thereby storing energy. At some point, the twisting or rotating force is released and the rotary lock spring may then be configured for unwinding and releasing the stored energy of the spring. This may provide that the one or more first male components become fully engaged with or completely inserted into the one or more first female components. In other words, the rotary lock spring may provide that the one or more first male components "snaps" into a final resting position of the one or more first female components, the final resting position being an end point of one or more first female components. The rotary lock spring may provide that manual rotation movement from the user is not needed for releasably locking the cartridge holder to the main body. This is an advantage because it provides an injection device that is easier to handle and easier to lock, especially for users with impaired strength.

In some embodiments, the piston rod assembly further comprises a piston washer. Thus, in other words, the injection device may comprise the piston washer. The piston rod assembly may further comprises a piston washer lock. Thus, in other words, the injection device may comprise the piston washer lock.

In some embodiments, the one or more first male components or one or more first female components provided at the rotary lock are configured for engaging with the one or more first male components or one or more first female components provided at the cartridge holder as the rotary lock rotates or when the rotary lock has rotated, such as around the longitudinal axis.

In some embodiments, the piston rod is cylindrical in shape and is configured to be provided inside the main body and/or the cartridge holder. When the main body and the cartridge holder are separated, the piston rod may be configured to be mostly or entirely provided inside the main body. When the main body and the cartridge holder are releasably locked, the piston rod may be configured for initially being provided mostly or entirely provided inside the main body. But as drug is being delivered from the cartridge, the piston rod may also be configured for being provided in the cartridge holder. The drive mechanism may further comprise a piston rod drive element. Thus, in other words, the injection device may comprise the piston rod drive element. The piston rod may comprise one or more longitudinal grooves. The piston rod drive element may comprise one or more drive element pins or protrusions configured such that the piston rod drive element is configured for engaging with the piston rod.

In some embodiments, the one or more first male components are further configured for being inserted into the one or more first female components. Engaging the one or more first male components with the one or more first female components may comprise rotating, twisting and/or moving the one or more first male components into the one or more first female components, such as around the longitudinal axis, in an clockwise or anti-clockwise direction, which is an opposite direction than unlocking. Thus, inserting may also comprise rotating, twisting and/or moving the one or more first male components into the one or more first female components. This is an advantage as it may provide a strong locking mechanism.

In some embodiments, the one or more first male components are further configured for disengaging from the one or more first female components. Disengaging the one or more first male components from the one or more first female components may comprise rotating, twisting and/or moving the one or more first male components out of the one or more first female components, such as around the longitudinal axis, in an clockwise or anti-clockwise direction, which is an opposite direction than locking. This is an advantage as it may provide easily unlocking.

In some embodiments, the one or more first male components are one or more pins or protrusions, and the one or more first female components are one or more tracks, slots, sockets, groves or indentations. The one or more first female components may comprise an elongated, nonlinear and/or curved shape. The one or more first female components may comprise a final resting position for the one or more first male components, the final resting position being an end point of one or more first female components which is provided opposite an entry point of the one or more tracks, slots, sockets, groves or indentations. The one or more first male components and the one or more first female components may be one or bayonet locks.

In some embodiments, the one or more first male components are one or more external threads, and the one or more first female components are one or more internal threads, such as a screw lock.

In some embodiments, the lock assembly further comprises a base bushing configured for connecting the cartridge holder to the main body. Thus, in other words the injection device comprises the base bushing. The lock assembly may further comprise one or more second male components and one or more second female components. The one or more second male components may be provided at the base bushing and the one or more second female components may be provided at the cartridge holder, or vice versa. The one or more second male and female components may be different from the one or more first male and female components. The one or more second male components may be configured for engaging with the one or more second female components. This is an advantage because the one or more second male components and the one or more second components may act as a guide to ensure that the cartridge holder and main body are aligned or correctly positioned relative to each other prior to and during the locking mechanism.

In some embodiments, the one or more second male or one or more second female components provided at the cartridge holder are provided at an outside surface or at an inside surface of the cartridge holder.

In some embodiments, the one or more second male components are further configured for aligning with the one or more second female components. Engaging the one or more second male components with the one or more second female components may comprise moving or sliding the one or more second male components into the one or more second female components, such as without rotating or twisting.

In some embodiments, the rotary lock, the rotary lock spring, the base bushing, and the piston rod drive element are provided in the main body. The rotary lock may be configured for being provided in the base bushing. The rotary lock may be further configured for rotational movement around the longitudinal axis. The rotary lock may be limited/restricted in any translatory movement by the base bushing and the piston rod drive element. This may be provided by a protrusion provided in the base bushing and/or a ball bearing provided in or adjacent the piston drive element.

In some embodiments, the injection device further comprises a battery, such as a rechargeable battery. In some embodiments, the injection device further comprises a microprocessor, such as a CPU, such as a CISC, RISC or EPIC.

In some embodiments, the injection device further comprise a push button. The push button may be configured for initiating delivering set doses of the drug from the cartridge. The injection device may further configured for providing that the battery can recharge. The injection device may further be configured for providing that the push button is prohibited from functioning when the battery is recharging. The injection device may further comprises a female charging connector configured for receiving a male charging connector for recharging the battery.

In some embodiments, the push button is prohibited from functioning by mechanical means. In some embodiments, the push button is prohibited from functioning by electronic means. In some embodiments, the push button is prohibited from functioning by moving, shifting or repositioning at least part of the start button. In some embodiments, the push button is an electronic push button comprising a push button sensor and a push button surface. When the push button is functioning, the push button sensor and the push button surface may be in contact with each other, and the push button sensor may be configured for providing an push button sensor output. The push button may be prohibited from functioning by moving, shifting or repositioning the push button surface relative to the push button sensor. Thus, when prohibiting the push button from functioning, the contact between the push button sensor and the push button surface may be broken. The injection may further comprise an end cover. The end cover may be provided adjacent to or together with or form part of the push button. The end cover may be configured for prohibiting contact with the push button sensor when the push button surface is repositioned relative to the push button sensor. This is an advantage because it prevent the user for accidentally touching or making contact with the push button sensor while charging the injection device and/or while repositioning the push button surface relative to the push button sensor.

In some embodiments, the injection device further comprises a housing configured for housing the main body. Hereby, the main body may be protected. The housing may have opening configured for revealing a display or other features of the main body or device. The injection device may further comprise a dose selector, such as a rotatable dose selector. The injection device may further comprise a base for the dose selector. The dose selector and/or base for the dose selector may be provided adjacent to or together with or form part of the push button.

In some embodiments, the drive mechanism comprises two or more electric motors. Thus, the at least one electric motor may be two or more motors. In other words, instead of having one motor, the drive mechanism may comprise two or more motors that are configured to work together, providing a cumulative effect. The two or more electric motors may have similar or different size, capabilities, power output and/or efficiency. It is an advantage that the required motor-power may be split up into two or more motors, because it provides than a compact injection device may be provided with more flexibility in designing the device and may provide a more compact injection while still maintaining the required motor-power. It is also an advantage that it may be the same two or more motors that may provide that the cartridge holder is unlocked from the main body, and may also further provide that drug is delivered from the cartridge.

In some embodiments, the drive mechanism further comprises a mechanical gear unit in connection with the at least one electric motor. Typically an electric motor will provide a relatively high speed and a small torque on a motor shaft. It is an advantage that the mechanical gear unit may provide speed reduction or torque increase, because the mechanical gear unit reduces the number of revolutions and increases the torque of the motor shaft. The mechanical gear unit may comprise one, two, three or four gears. In some embodiments, the mechanical gear unit comprises two or more gears, and the injection device further comprises a first gear unit sensor and a second gear unit sensor, such as tacho sensor or rotary position sensor. The first gear unit sensor may be provided before a first gear of the mechanical gear unit, and the second gear unit sensor may be provided after a final gear of the mechanical gear unit. Thus, the first and second gear unit sensors may be provided at opposite ends of the mechanical gear unit. The first and second gear unit sensors may be configured for sensing a rotary position of the first and final gears, respectively. Hereby, a comparison of an output from the first and second gear unit sensors is possible and this is an advantage because it may provide performance monitoring of the mechanical gear unit.

In some embodiments, the first gear unit sensor and the second gear unit sensor are configured for providing a first gear unit sensor output and a second gear unit sensor output, respectively. The first and second gear unit sensor outputs may be configured for measuring an elastic deformation of the mechanical gear unit. This is an advantage because it may be used for performance monitoring and/or measuring backpressure from the cartridge to the piston rod assembly after end dose.

In some embodiments, a power consumption of the at least one electric motor is measured. The power consumption of the at least one electric motor and the elastic deformation of the mechanical gear unit may be used for determining a degree of deterioration or wear of tear of the mechanical gear unit.

In some embodiments, the injection device further comprises a cap for protecting the cartridge. The cap may be configured for receiving the cartridge holder. The cap may be configured for being releasably attached to the cartridge holder. The cap may comprise a cap tube and/or a clip.

In some embodiments, the injection device further comprises at least one cap sensor. The at least one cap sensor may be provided at the main body. The at least one cap sensor may be configured for sensing when the cap is releasably attached to the cartridge holder. The at least one cap sensor may be configured for providing a cap sensor output. The cap sensor output may be digital output. The at least one cap sensor may be a switch sensor, a spring contact sensor or a photoelectric sensor. The switch sensor may be configured for either opening or closing an electronic circuit, such as a normal intended circuit for electric charge between the battery and the microprocessor. The photoelectric sensor may be configured for using light to detect that the cap is releasably attached to the main body. If the cap is releasably attached to the main body, the device is not able to deliver set doses of the drug from the cartridge. Thereby, there is no need for the battery to deliver power to the microprocessor when the cap is releasably attached to the main body. The cap sensor output may provide that the microprocessor and the battery becomes electrically disconnected. Alternatively, the cap sensor output may provide that the microprocessor is still electrically connected to the battery, but provides that the microprocessor is in a power saving mode or sleep mode, which is configured for reducing power consumption. Thereby, this is an advantage because it preserves battery power while the user is still able to interact with the injection device if needed.

In some embodiments, the at least one sensor is a spring contact sensor comprising a cap sensor element, a cap sensor spring and a cap sensor terminal. The cap sensor element may be configured to be shifted from a first position to a second position when the cap is being releasably attached to the cartridge holder. The cap sensor element in the second position may be configured to manipulate the cap sensor spring such that the cap sensor spring and the cap sensor terminal provides an electrical connection and provides the cap sensor output.

In some embodiments, the cartridge holder comprises a first magnetic element and the cap comprises a second magnetic element, the first and the second magnetic elements are configured to being attracted to each other. The first magnetic element may be a magnet or a magnetic material. The second magnetic element may be a magnet or a magnetic material. Magnetic material may be magnetic metals, such as transition metals, or magnetic compounds, ceramic magnets or magnetic alloys. This is an advantage because it provides that the cap may easily snap onto the cartridge holder.

In some embodiments, the first magnetic element is provided in a first end of the cartridge holder and the second magnetic element is provided in a first end of the cap. Alternatively, the first magnetic element may be provided in a second end of the cartridge holder and the second magnetic element may be provided in a second end of the cap. The second end of the cap being provided opposite to the first end of the cap, and the second end of the cartridge holder being provided opposite to the first end of the cartridge holder.

In some embodiments, the first magnetic element comprises a plurality of elements, such as an array of elements, such as more than one element, and the second magnetic element comprises a plurality of elements, such as an array of elements, such as more than one element. The first and/or second magnetic element may be in the shape of a ring.

In some embodiments, the injection device further comprises one or more output components, the output components are configured for providing a haptic, tactile, audio and/or visual output. The one or more output components may be e.g. vibration motors or tactile transducers for haptic/tactile output and/or audio transducer/speaker for audio output.

In some embodiments, the injection device further comprises a dose selector. The one or more output components my be configured for providing the output in response to the user operating the dose selector. Alternatively or additionally, the one or more output components may be configured for providing the output in response a determination that the user is due to receive a set dose of the drug from the cartridge. Alternatively, or additionally, the one or more output components may be configured for providing the output in response to a determination that a set dose of drug from the cartridge has been fully or completely administrated. Alternatively, or additionally, one of the one or more output components may be a display configured for providing a visual output, and the display may be configured for providing the visual output in response to user input via the push button.

In some embodiments, the injection device further comprises an accelerometer configured for measuring acceleration and configured for providing an accelerometer output. Alternatively, or additionally, in some embodiments, the injection device further comprises a gyroscope or gyrometer configured for measuring orientation and/or angular position and further configured for providing an gyroscope output. The gyroscope may be a micro-chip-packaged or microelectromechanical (MEMS) gyroscope. The accelerometer may be a micro-chip-packaged or microelectromechanical (MEMS) accelerometer.

The microprocessor may be configured for receiving the accelerometer output and/or gyroscope output.

Also disclosed is a system. The system comprises an injection device according to the above disclosure. The system further comprises an electronic device such as a smartphone. The electronic device is configured for wired or wireless connection with the injection device. The system further comprises an external server. The external server is configured for wired or wireless connection with the injection device and/or the electronic device. The injection device further comprises an accelerometer configured for measuring acceleration and configured for providing an accelerometer output, and/or the injection device further comprises a gyroscope configured for measuring orientation and/or angular position and further configured for providing an gyroscope output. The microprocessor is configured for receiving the accelerometer output and/or gyroscope output. The injection device is further configured for transmitting the accelerometer output and/or gyroscope output, to the electronic device or the external server for data processing, such as further data processing. This is an advantage because the accelerometer output and/or gyroscope output may provide performance monitoring.

In some embodiments, the user is notified, via the electronic device or via the injection device, if the accelerometer output and/or gyroscope output indicates that the injection device is in risk of malfunctioning and/or providing incorrect doses. The user may be notified via the one or more output components.

Also disclosed is an injection device set comprising an injection device according to above disclosure and a cap according to the above disclosure.

Thus, the cap may be considered part of the injection device, or alternatively, part of the injection device set.

The present invention relates to different aspects including the injection device described above and in the following, and the disclosed system and set, each yielding one or more of the benefits and advantages described in connection with the first mentioned aspect, and each having one or more embodiments corresponding to the embodiments described in connection with the first mentioned aspect and/or disclosed in the appended claims.

### BRIEF DESCRIPTION OF THE DRAWINGS

The above and other features and advantages will become readily apparent to those skilled in the art by the following detailed description of exemplary embodiments thereof with reference to the attached drawings, in which:
Fig. 1 shows an exploded-view drawing of an exemplary injection device.
Fig. 2a shows an exploded-view drawing of an exemplary injection device.
Fig. 2b shows an cross-sectional drawing of an exemplary injection device.
Fig. 3a and Fig. 3b show exploded-view drawings of an exemplary device.
Fig. 3c shows a partial cross-section drawing of an exemplary device.
Fig. 4a shows an exploded-view drawing of an exemplary device.
Fig. 4b and Fig. 4c show an cross-sectional drawing of an exemplary injection device.
Fig. 5 shows an exploded-view drawing of an exemplary device.
Fig. 6 and Fig. 6b show an exemplary device.

### DETAILED DESCRIPTION

Various embodiments are described hereinafter with reference to the figures. Like reference numerals refer to like elements throughout. Like elements will, thus, not be described in detail with respect to the description of each figure. It should also be noted that the figures are only intended to facilitate the description of the embodiments.

They are not intended as an exhaustive description of the claimed invention or as a limitation on the scope of the claimed invention. In addition, an illustrated embodiment needs not have all the aspects or advantages shown. An aspect or an advantage described in conjunction with a particular embodiment is not necessarily limited to that embodiment and can be practiced in any other embodiments even if not so illustrated, or if not so explicitly described.

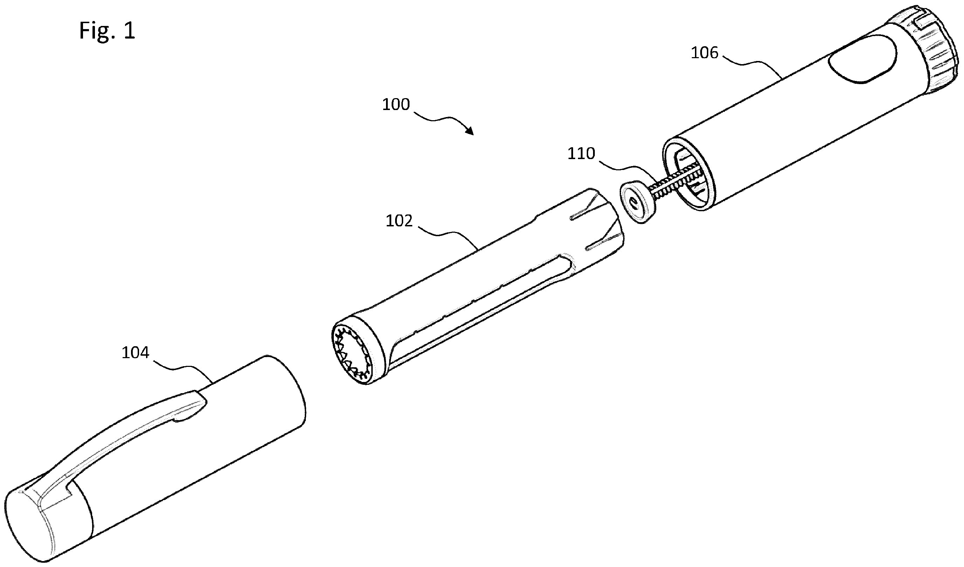

Fig. 1 shows an exploded-view drawing of an exemplary injection device 100 for delivering set doses of a drug from a cartridge (not shown). The injection device 100 comprises a main body 106. The injection device 100 comprises a cartridge holder 102 configured for holding the cartridge. The injection device 100 comprises a piston rod assembly comprising a piston rod 110. The injection device comprises a lock assembly. The lock assembly is configured for releasably locking the cartridge holder 102 to the main body 106. The injection device 100 comprises a drive mechanism. The drive mechanism is configured for driving the lock assembly to unlock the cartridge holder 102 from the main body 106. The drive mechanism comprises at least one electric motor (not shown).

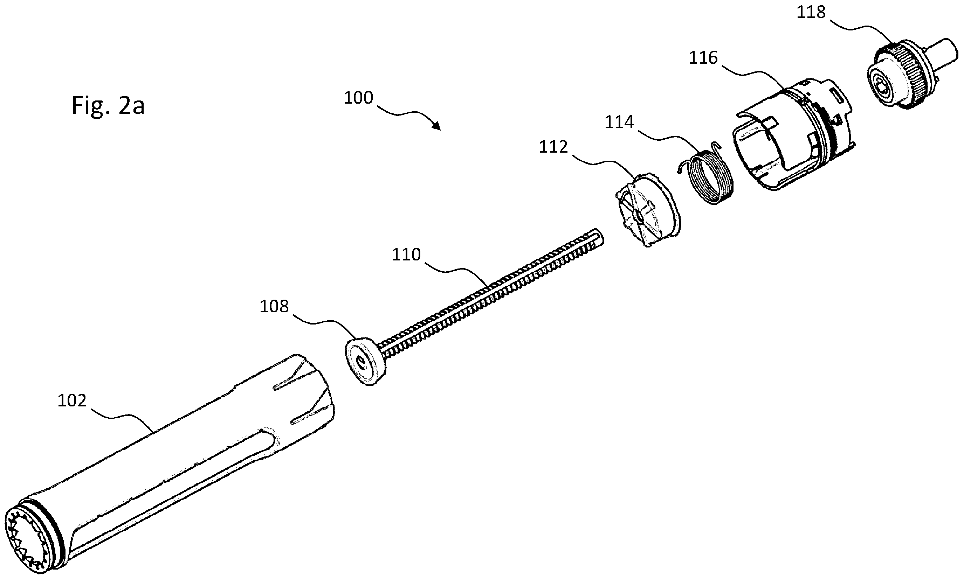

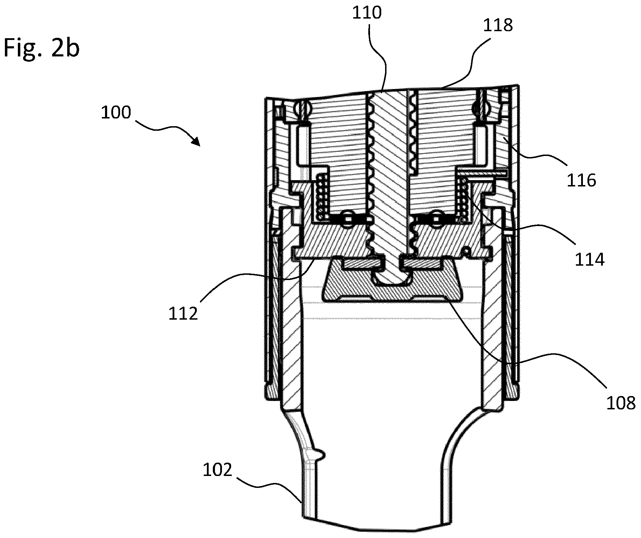

Fig. 2a shows an exploded-view drawing of an exemplary injection device 100 for delivering set doses of a drug from a cartridge (not shown). The injection device 100 comprises a main body, however, this feature is not shown in figure 2 (see figure 1 for main body). The injection device 100 comprises a cartridge holder 102. The injection device 100 is further shown to comprise a rotary lock 112, a rotary lock spring 114, a base bushing 116, and a piston rod drive element 118, all provided in the main body (not shown). The base bushing 116 is configured for connecting the cartridge holder 102 to the main body (not shown). The rotary lock spring 114 is configured for providing energy to support releasably locking the cartridge holder 102 to the main body (not shown). The piston rod drive element 118 is configured for engaging with the piston rod 110. The injection device 100 comprises a piston rod assembly, the piston rod assembly comprises a piston rod 110. The piston rod assembly comprises a piston washer 108.

Fig. 2b shows an cross-sectional drawing of an exemplary injection device 100 for delivering set doses of a drug from a cartridge (not shown). For illustrative purposes, only the features shown in Fig. 2a is mentioned here. Thus, the injection device 100 comprises a cartridge holder 102, a piston rod 110, a piston washer 108, a rotary lock 112, a rotary lock spring 114 and a base bushing 116.

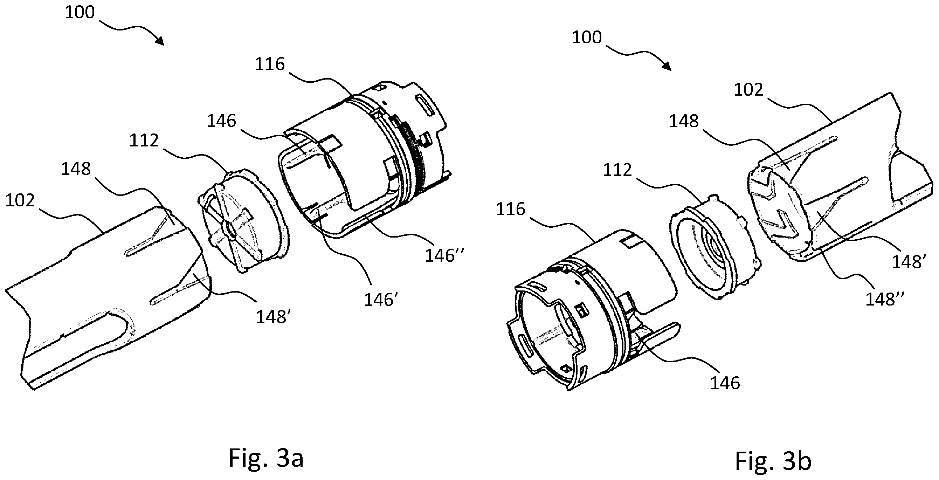

Fig. 3a and Fig. 3b show exploded-view drawings of an exemplary device 100 for delivering set does of a drug from a cartridge (not shown). Fig. 3a and Fig. 3b shows the injection device from two different viewing angles. The injection device 100 comprises a cartridge holder 102, a rotary lock 112 and a base bushing 116. The injection device 100 comprises a lock assembly that comprises one or more second male components 146 and one or more second female components 148, the one or more second male components 146 are configured for engaging with the one or more second female components 148.

In Fig. 3a and Fig. 3b, the one or more second male components 146 are provided at the base bushing 116 and the one or more second female components 148 are provided at the cartridge holder 102. Alternatively, the one or more second male components 146 may be provided at the cartridge holder 102 and the one or more second female components 148 may be provided at the base bushing 116. The one or more second female components 148 are illustrated as being provided on an outside surface of the cartridge holder 102.

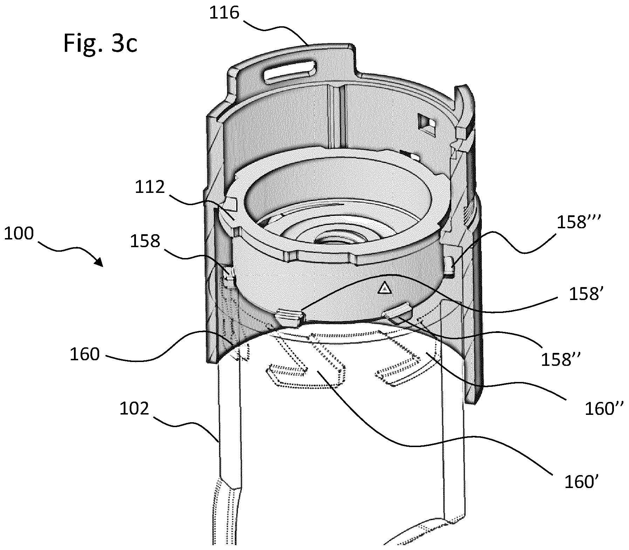

Fig. 3c shows a partial cross-section drawing of an exemplary device 100 for delivering set does of a drug from a cartridge (not shown). The injection device 100 comprises a cartridge holder 102, a rotary lock 112 and a base bushing 116. The injection device 100 comprises a lock assembly that comprises one or more first male components 158 and one or more first female components 160, the one or more first male components 158 are configured for engaging with the one or more first female components 160. In the figure, the one or more first male components 158 are provided at the rotary lock 112 and the one or more first female components 160 are provided at the cartridge holder 102. Alternatively, the one or more first male components 158 may be provided at the cartridge holder 102 and the one or more first female components 160 may be provided at the rotary lock 112. The one or more first female components 160 are illustrated as being provided at an inside surface of the cartridge holder 102.)

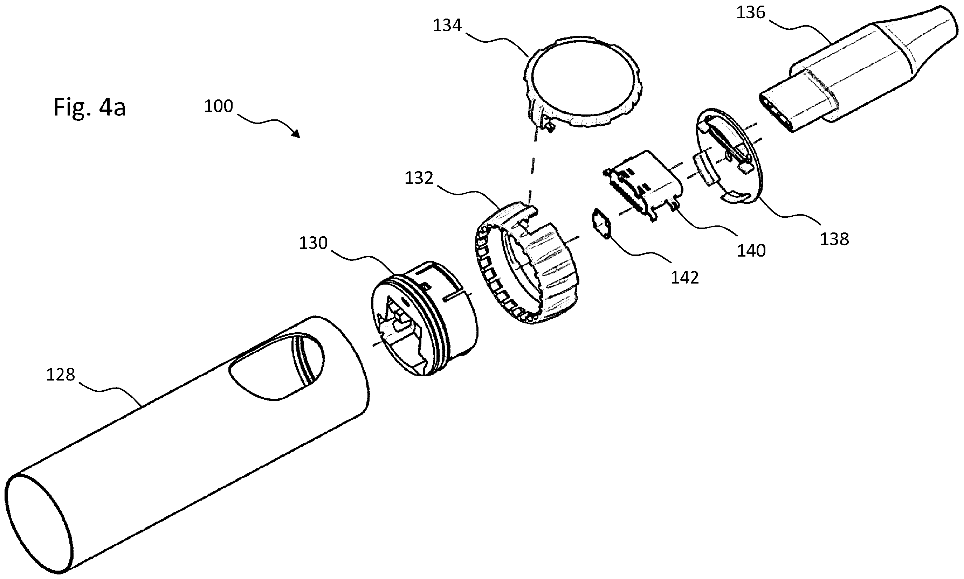

Fig. 4a shows an exploded-view drawing of an exemplary device 100 for delivering set does of a drug from a cartridge (not shown). In some embodiments, the injection device 100 further comprises a battery (not shown), such as a rechargeable battery.

The injection device 100 may further comprise a push button. The push button may be configured for initiating delivering set doses of the drug from the cartridge. The injection device 100 may further configured for providing that the battery (not shown) can recharge. The injection device 100 100 may further be configured for providing that the push button is prohibited from functioning when the battery (not shown) is recharging. The injection device 100 comprises a female charging connector 140 configured for receiving a male charging connector 136 for recharging the battery (not shown).

In the figure, the push button is an electronic push button comprising a push button sensor 142 and a push button surface 134. When the push button is functioning, the push button sensor 142 and the push button surface 134 may be in contact with each other, and the push button sensor may be configured for providing an push button sensor output. The push button may be prohibited from functioning by moving, shifting or repositioning the push button surface 134 relative to the push button sensor 142.

In the figure, the injection device further comprises a housing 128, a dose selector 132, a base for the dose selector 132 and an end cover 138. The end cover is configured for prohibiting contact with the push button sensor 142 when the push button surface 134 is repositioned relative to the push button sensor 142.

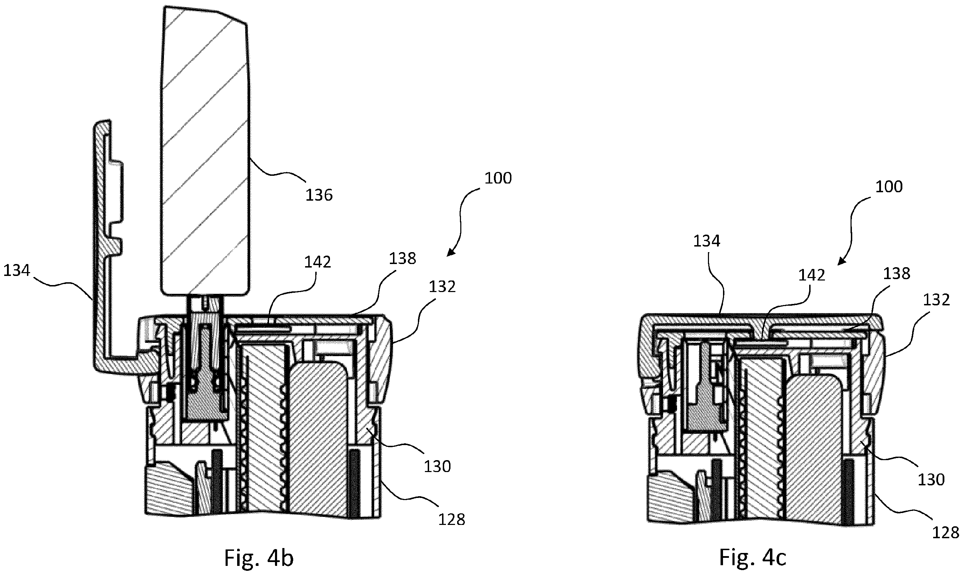

Fig. 4b and Fig. 4c show an cross-sectional drawing of an exemplary injection device1 00 for delivering set doses of a drug from a cartridge (not shown). For illustrative purposes, only the features shown in Fig. 4a is mentioned here. Fig. 4c shows that the push button sensor 142 and the push button surface 134 are in contact with each other. Fig. 4b shows the male charging connector 136 inserted into the female charging connector and that the push button is prohibited from functioning because the push button surface 134 is repositioned relative to the push button sensor 142.

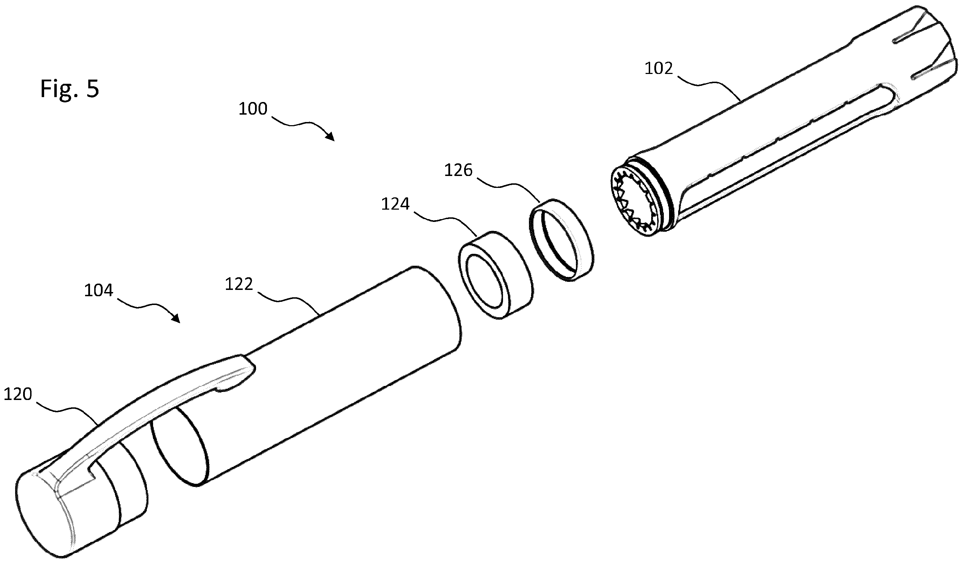

Fig. 5 shows an exploded-view drawing of an exemplary device 100 for delivering set does of a drug from a cartridge (not shown). The injection device 100 may be part of an injection device set. The injection device 100 comprises a cartridge holder 102. The injection device 100 or the injection device set comprises a cap 104 for protecting the cartridge. The cap 104 is configured for receiving the cartridge holder 102. The cap 104 is configured for being releasably attached to the cartridge holder 102. The cap 104 is shown to comprise a cap tube 122 and a clip 120.

The cartridge holder 102 comprises a first magnetic element 126 and the cap 104 comprises a second magnetic element 124. The first and the second magnetic elements 124, 126 are configured to being attracted to each other.

In the figure, the first magnetic element 126 is provided in a first end of the cartridge holder 102 and the second magnetic element 124 is provided in a first end of the cap 104 or cap tube 122. Alternatively, the first magnetic element 126 may be provided in a second end of the cartridge holder 102 and the second magnetic element 124 may be provided in a second end of the cap 104 or cap tube 122. The second end of the cap 104 or cap tube 122 being provided opposite to the first end of the cap 104 or cap tube 122, and the second end of the cartridge holder 102 being provided opposite to the first end of the cartridge holder 102.

In the figure, the first and second magnetic element 124, 126 are shown in the shape of a ring. Alternatively, the first and/or second magnetic element 124, 126 may comprise a plurality of elements.

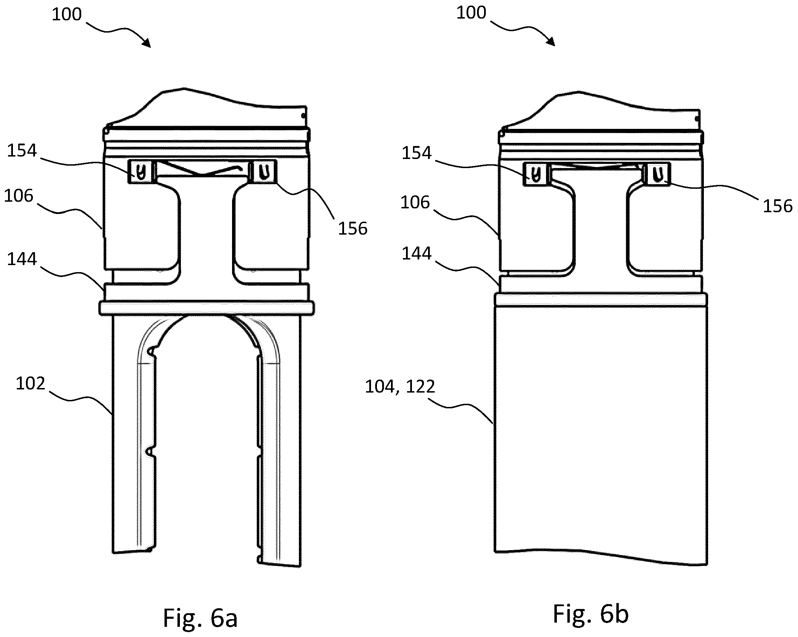

Fig. 6a and Fig. 6b show an exemplary device 100 for delivering set does of a drug from a cartridge (not shown). The injection device 100 may be part of an injection device set. The injection device comprises a main body 106 and a cartridge holder 102. The injection device 100 or the injection device set may comprise a cap 104 comprising a cap tube 122. The figure shows only part of the injection device 100, focusing on an area where the cartridge holder 102 is configured for being connected to the main body 106. Fig. 6a shows the injection device 100 without the cap 104. Fig. 6b shows the cap 104 being releasably attached to the cartridge holder 102.

The injection device comprises at least one cap sensor, the at least one cap sensor is provided at the main body 106. The at least one cap sensor may be configured for sensing when the cap 104 is releasably attached to the cartridge holder 102. In the figure, the at least one cap sensor is a spring contact sensor comprising a cap sensor element 144, a cap sensor spring 154 and a cap sensor terminal 156. The cap sensor element 144 is configured to be shifted by the cap tube 122 from a first position (shown in Fig. 6a) to a second position (shown in Fig. 6b) when the cap 104 is being releasably attached to the cartridge holder 102. The cap sensor element 144 in the second position is shown to be configured to manipulate the cap sensor spring 154 such that the cap sensor spring 154 and the cap sensor terminal 156 provides an electrical connection and provides a cap sensor output.

Although particular features have been shown and described, it will be understood that they are not intended to limit the claimed invention, and it will be made obvious to those skilled in the art that various changes and modifications may be made without departing from the scope of the claimed invention. The specification and drawings are, accordingly to be regarded in an illustrative rather than restrictive sense. The claimed invention is intended to cover all alternatives, modifications and equivalents.

### ITEMS

1. An injection device for delivering set doses of a drug from a cartridge, the injection device comprising:
   a main body :
   a cartridge holder configured for holding the cartridge;
   a piston rod assembly comprising a piston rod;
   a lock assembly configured for releasably locking the cartridge holder to the main body; and a drive mechanism configured for driving the lock assembly to unlock the cartridge holder from the main body, wherein the drive mechanism comprises at least one electric motor.
2. The injection device according to item 2, wherein the drive mechanism is configured for driving the lock assembly to unlock the cartridge holder from the main body by driving the piston rod assembly to engage with the lock assembly.
3. The injection device according to any one of the preceding items, wherein the drive mechanism is further configured for driving the piston rod assembly to engage with the cartridge to deliver set doses of the drug.
4. The injection device according to any one of the preceding items, wherein the lock assembly comprises one or more first male components and one or more first female components, the one or more first male components are configured for engaging with the one or more first female components.
5. The injection device according to any one of the preceding items, wherein the lock assembly further comprises a rotary lock, wherein the one or more first male components are provided at the rotary lock and wherein the one or more first female components are provided at the cartridge holder, or vice versa.
6. The injection device according to any one of the preceding items, wherein the piston rod and the rotary lock comprises external threads and internal threads, respectively, and wherein the piston rod assembly engaging with the lock assembly comprises rotating the rotary lock.
7. The injection device according to any one of the preceding items, wherein the one or more first male components or one or more first female components provided at the cartridge holder are provided at an inside surface, at an outside surface or at an boundary/edge between the inside and outside surface of the cartridge holder.
8. The injection device according to any one of the preceding items, wherein an longitudinal axis of the injection device runs lengthwise through a centre of the injection device, and wherein the rotary lock rotates around the longitudinal axis.
9. The injection device according to any one of the preceding items, wherein the lock assembly further comprises a rotary lock spring configured for providing energy to support releasably locking the cartridge holder to the main body, wherein the rotary lock spring is a torsion-spring, such as a helical torsion spring.
10. The injection device according to any one of the preceding items, wherein the piston rod assembly further comprises a piston washer and a piston washer lock.
11. The injection device according to any one of the preceding items, wherein the one or more first male components or the one or more first female components provided at the rotary lock are configured for engaging with the one or more first male components or the one or more first female components provided at the cartridge holder as the rotary lock rotates or when the rotary lock has rotated.
12. The injection device according to any one of the preceding items, wherein the piston rod is cylindrical in shape and is configured to be provided inside the main body and/or the cartridge holder, wherein the drive mechanism further comprises a piston rod drive element, wherein the piston rod comprises one or more longitudinal grooves, and wherein the piston rod drive element comprises one or more drive element pins or protrusions configured such that the piston rod drive element is configured for engaging with the piston rod.
13. The injection device according to any one of the preceding items, wherein the one or more first male components are further configured for being inserted into the one or more first female components, and wherein engaging the one or more first male components with the one or more first female components comprises rotating, twisting and/or moving the one or more first male components into the one or more first female components.
14. The injection device according to any one of the preceding items, wherein the one or more first male components are further configured for disengaging from the one or more first female components, wherein disengaging the one or more first male components from the one or more first female components comprises rotating, twisting and/or moving the one or more first male components out of the one or more first female components.
15. The injection device according to any one of the preceding items, wherein the one or more first male components are one or more pins or protrusions, and wherein the one or more first female components are one or more tracks, slots, sockets, groves or indentations, and wherein the one or more first female components comprises an elongated, nonlinear and/or curved shape.
16. The injection device according to any one of the preceding items, wherein the one or more first male components are one or more external threads, and wherein the one or more first female components are one or more internal threads.
17. The injection device according to any one of the preceding items, wherein the lock assembly further comprises a base bushing configured for connecting the cartridge holder to the main body, wherein the lock assembly further comprises one or more second male components and one or more second female components, wherein the one or more second male components are provided at the base bushing and the one or more second female components are provided at the cartridge holder, or vice versa, and wherein the one or more second male components are configured for engaging with the one or more second female components.
18. The injection device according to any one of the preceding items, wherein the one or more second male or one or more second female components provided at the cartridge holder are provided at an outside surface or at an inside surface of the cartridge holder.
19. The injection device according to any one of the preceding items, wherein the one or more second male components are further configured for aligning with the one or more second female components, and wherein engaging the one or more second male components with the one or more second female components comprises moving or sliding the one or more second male components into the one or more second female components.
20. The injection device according to any one of the preceding items, wherein the rotary lock, the rotary lock spring, the base bushing, and the piston rod drive element are provided in the main body.
21. The injection device according to any one of the preceding items, wherein the rotary lock is configured for being provided in the base bushing, wherein the rotary lock is further configured for rotational movement around the longitudinal axis, and wherein the rotary lock is limited/restricted in any translatory movement by the base bushing and the piston rod drive element.
22. The injection device according to any one of the preceding items, wherein the injection device further comprises a battery and a microprocessor, wherein the injection device further comprises a push button configured for initiating delivering set doses of the drug from the cartridge, wherein the injection device is further configured for providing that the battery can recharge, and wherein the injection device is further configured for providing that the push button is prohibited from functioning when the battery is recharging.
23. The injection device according to any one of the preceding items, wherein the injection device further comprises a female charging connector configured for receiving a male charging connector for recharging the battery.
24. The injection device according to any one of the preceding items, wherein the push button is prohibited from functioning by mechanical means.
25. The injection device according to any one of the preceding items, wherein the push button is prohibited from functioning by electronic means.
26. The injection device according to any one of the preceding items, wherein the push button is prohibited from functioning by moving, shifting or repositioning at least part of the start button.
27. The injection device according to any one of the preceding items, wherein the push button is an electronic push button comprising a push button sensor and a push button surface, and wherein the push button is prohibited from functioning by moving, shifting or repositioning the push button sensor relative to the push button surface.
28. The injection device according to any one of the preceding items, wherein the drive mechanism comprises two or more electric motors.
29. The injection device according to any one of the preceding items , wherein the two or more electric motors have similar or different size, capabilities, power output and/or efficiency.
30. The injection device according to any one of the preceding items, wherein the drive mechanism further comprises a mechanical gear unit in connection with the at least one electric motor.
31. The injection device according to any one of the preceding items, wherein the mechanical gear unit comprises one, two, three, four gears.
32. The injection device according to any one of the preceding items, wherein the mechanical gear unit comprises two or more gears, and wherein the injection device further comprises a first gear unit sensor and a second gear unit sensor, wherein the first gear unit sensor is provided before a first gear of the mechanical gear unit, and wherein the second gear unit sensor is provided after a final gear of the mechanical gear unit, and wherein the first and second gear unit sensors are configured for sensing a rotary position of the first and final gears, respectively.
33. The injection device according to any one of the preceding items, wherein the first gear unit sensor and the second gear unit sensor are configured for providing a first gear unit sensor output and a second gear unit sensor output, respectively, and wherein the first and second gear unit sensor outputs are configured for measuring an elastic deformation of the mechanical gear unit.
34. The injection device according to any one of the preceding items, wherein a power consumption of the at least one electric motor is measured, and wherein the power consumption of the at least one electric motor and the elastic deformation of the mechanical gear unit is used for determining a degree of deterioration of the mechanical gear unit.
35. A system comprising the injection device according to any one of the preceding claims, the system further comprises an electronic device such as a smartphone, the electronic device configured for wired or wireless connection with the injection device , the system further comprises an external server, the external server configured for wired or wireless connection with the injection device and/or the electronic device.
36. The injection device according to any one of the preceding items, wherein the injection device further comprises an accelerometer configured for measuring acceleration and configured for providing an accelerometer output, and/or wherein the injection device further comprises a gyroscope configured for measuring orientation and/or angular position and further configured for providing an gyroscope output, and wherein microprocessor is configured for receiving the accelerometer output and/or gyroscope output, and wherein the injection device is further configured for transmitting the accelerometer output and/or gyroscope output, to the electronic device or the external server for data processing, such as further data processing.
37. The injection device according to any one of the preceding items, wherein the user is notified, via the electronic device or via the injection device , if the accelerometer output and/or gyroscope output indicates that the injection device is in risk of malfunctioning and/or providing incorrect doses.
38. An injection device set comprising an injection device according to any one of the preceding claims and a cap, the cap is configured for receiving the cartridge holder, the cap is configured for being releasably attached to the cartridge holder.
39. The injection device according to any one of the preceding items, wherein the injection device further comprises a cap for protecting the cartridge, the cap is configured for receiving the cartridge holder, the cap is configured for being releasably attached to the cartridge holder.
40. The injection device according to any one of the preceding items or the injection device set according to any one of the preceding items, wherein the injection device further comprises at least one cap sensor, the at least one cap sensor is provided at the main body, wherein the at least one cap sensor is configured for sensing when the cap is releasably attached to the cartridge holder.
41. The injection device according to any one of the preceding items or the injection device set according to any one of the preceding items, wherein the at least one cap sensor may be a switch sensor, a spring contact sensor or a photoelectric sensor.
42. The injection device according to any one of the preceding items or the injection device set according to any one of the preceding items, wherein the at least one sensor is a spring contact sensor comprising a cap sensor element, a cap sensor spring and a cap sensor terminal, wherein the cap sensor element is configured to be shifted from a first position to a second position when the cap is being releasably attached to the cartridge holder, wherein the cap sensor element in the second position is configured to manipulate the cap sensor spring such that the cap sensor spring and the cap sensor terminal provides a short circuit.
43. The injection device according to any one of the preceding items or the injection device set according to any one of the preceding items, wherein the cartridge holder comprises a first magnetic element and wherein the cap comprises a second magnetic element, the first and the second magnetic elements are configured to being attracted to each other.
44. The injection device according to any one of the preceding items or the injection device set according to any one of the preceding items, wherein the first magnetic element is a magnet or a magnetic material, and wherein the second magnetic element is a magnet or a magnetic material.
45. The injection device according to any one of the preceding items or the injection device set according to any one of the preceding items, wherein the first magnetic element is provided in a first end of the cartridge holder and the second magnetic element is provided in a first end of the cap.
46. The injection device according to any one of the preceding items or the injection device set according to any one of the preceding items, wherein the first magnetic element comprises a plurality of elements, such as an array of elements, such as more than one element, and wherein the second magnetic element comprises a plurality of elements, such as an array of elements, such as more than one element.
47. The injection device according to any one of the preceding items or the injection device set according to any one of the preceding items, wherein the first and/or second magnetic element is in the shape of a ring.
48. The injection device according to any one of the preceding items, wherein the injection device further comprises one or more output components, the output components are configured for providing a haptic, tactile, audio and/or visual output.
49. The injection device according to any one of the preceding items, wherein the injection device further comprises a dose selector and wherein the one or more output components are configured for providing the output in response to the user operating the dose selector.
50. The injection device according to any one of the preceding items, wherein the one or more output components are configured for providing the output in response a determination that the user is due to receive a set dose of the drug from the cartridge.
51. The injection device according to any one of the preceding items, wherein the one or more output components are configured for providing the output in response to a determination that a set dose of drug from the cartridge has been fully or completely administrated.
52. The injection device according to any one of the preceding items, wherein one of the one or more output components is a display configured for providing a visual output, and wherein the display is configured for providing the visual output in response to user input via the push button.

### LIST OF REFERENCES

100 injection device
102 cartridge holder
104 cap
106 main body
108 piston washer
110 piston rod
112 rotary lock
114 rotary lock spring
116 base bushing
118 piston rod drive element
120 clip for cap
122 cap tube
124 cap magnet
126 cartridge holder magnet
128 housing
130 base for dose selector
132 dose selector
134 push button surface
136 male charging connector
138 end cover
140 female charging connector
142 push button sensor
144 cap sensor element
146 one or more second male components
148 one or more second female components
152 piston washer lock
154 cap sensor spring
156 cap sensor terminal
158 one or more first male components
160 one or more first female components

## Claims

1. An injection device (100) for delivering set doses of a drug from a cartridge, the injection device (100) comprising:
- a main body (106):
- a cartridge holder (102) configured for holding the cartridge;
- a piston rod assembly comprising a piston rod (110);
- a lock assembly configured for releasably locking the cartridge holder (102) to the main body (106); and
- a drive mechanism configured for driving the lock assembly to unlock the cartridge holder (102) from the main body (106), wherein the drive mechanism comprises at least one electric motor.

2. The injection device (100) according to claim 1, wherein the drive mechanism is further configured for driving the piston rod assembly to engage with the cartridge to deliver set doses of the drug.

3. The injection device (100) according to any one of the preceding claims, wherein the drive mechanism comprises two or more electric motors.

4. The injection device (100) according to any one of the preceding claims, wherein the lock assembly comprises one or more first male components (158) and one or more first female components (160), the one or more first male components (158) are configured for engaging with the one or more first female components (160).

5. The injection device (100) according to any one of the preceding claims, wherein the lock assembly further comprises a rotary lock (112), wherein the one or more first male components (158) are provided at the rotary lock (112) and wherein the one or more first female components (160) are provided at the cartridge holder (102), or vice versa.

6. The injection device (100) according to any one of the preceding claims, wherein the lock assembly further comprises a rotary lock spring (114) configured for providing energy to support releasably locking the cartridge holder (102) to the main body (106), wherein the rotary lock spring (114) is a torsion-spring, such as a helical torsion spring.

7. The injection device (100) according to any one of the preceding claims, wherein the lock assembly further comprises a base bushing (116) configured for connecting the cartridge holder (102) to the main body (106), wherein the lock assembly further comprises one or more second male components (146) and one or more second female components (148), wherein the one or more second male components (146) are provided at the base bushing (116) and the one or more second female components (148) are provided at the cartridge holder (102), or vice versa, and wherein the one or more second male components (146) are configured for engaging with the one or more second female components (148).

8. The injection device (100) according to any one of the preceding claims, wherein the injection device (100) further comprises a battery and a microprocessor, wherein the injection device (100) further comprises a push button configured for initiating delivering set doses of the drug from the cartridge, wherein the injection device (100) is further configured for providing that the battery can recharge, and wherein the injection device (100) is further configured for providing that the push button is prohibited from functioning when the battery is recharging.

9. The injection device (100) according to any one of the preceding claims, wherein the push button is an electronic push button comprising a push button sensor (142) and a push button surface (134), and wherein the push button is prohibited from functioning by moving, shifting or repositioning the push button surface (134) relative to the push button sensor (142).

10. The injection device (100) according to any one of the preceding claims, wherein the drive mechanism further comprises a mechanical gear unit in connection with the at least one electric motor, wherein the mechanical gear unit comprises two or more gears, and wherein the injection device (100) further comprises a first gear unit sensor and a second gear unit sensor, wherein the first gear unit sensor is provided before a first gear of the mechanical gear unit, and wherein the second gear unit sensor is provided after a final gear of the mechanical gear unit, and wherein the first and second gear unit sensors are configured for sensing a rotary position of the first and final gears, respectively.

11. The injection device (100) according to any one of the preceding claims, wherein the injection device (100) further comprises a cap (104) for protecting the cartridge, wherein the cap (104) is configured for receiving the cartridge holder (102), and wherein the cap is configured for being releasably attached to the cartridge holder (102).

12. The injection device (100) according to any one of the preceding claims, wherein the injection device (100) further comprises at least one cap sensor, wherein the at least one cap sensor is provided at the main body (106), and wherein the at least one cap sensor is configured for sensing when the cap (104) is releasably attached to the cartridge holder (102).

13. The injection device (100) according to any one of the preceding claims, wherein the cartridge holder (102) comprises a first magnetic element (126), wherein the cap (104) comprises a second magnetic element (124), and wherein the first and the second magnetic elements (126, 124) are configured to being attracted to each other.

14. The injection device (100) according to any one of the preceding claims, wherein the injection device (100) further comprises one or more output components, the output components are configured for providing a haptic, tactile, audio and/or visual output in response to user input and/or to notify the user.

15. A system comprising the injection device (100) according to any one of the preceding claims, the system further comprises:
- an electronic device such as a smartphone, the electronic device configured for wired or wireless connection with the injection device (100);
- an external server, the external server configured for wired or wireless connection with the injection device and/or the electronic device; and
wherein the injection device (100) further comprises an accelerometer configured for measuring acceleration and configured for providing an accelerometer output, and/or wherein the injection device (100) further comprises a gyroscope, the gyroscope configured for measuring orientation and/or angular position and further configured for providing an gyroscope output, wherein the microprocessor is configured for receiving the accelerometer output and/or gyroscope output, and wherein the injection device (100) is further configured for transmitting the accelerometer output and/or gyroscope output to the electronic device or the external server for data processing, such as further data processing.
